# EUROPÄISCHE PATENTSCHRIFT

(11) **EP 1 755 735 B1**
(45) Veröffentlichungstag und Bekanntmachung des Hinweises auf die Patenterteilung: **21.11.2018**
(21) Anmeldenummer: 05747699.6
(22) Anmeldetag: 23.04.2005
(51) Int. Cl.: A61N 1/36

(54) **VORRICHTUNG ZUR BEHANDLUNG VON PATIENTEN MITTELS HIRNSTIMULATION**
DEVICE FOR TREATING PATIENTS BY BRAIN STIMULATION
DISPOSITIF DE TRAITEMENT DE PATIENTS PAR STIMULATION DU CERVEAU

(30) Priorität: 24.05.2004 DE 102004025825
(43) Veröffentlichungstag der Anmeldung: 28.02.2007
(73) Patentinhaber: Forschungszentrum Jülich GmbH, 52425 Jülich (DE)
(72) Erfinder: TASS, Peter, 81541 München (DE)
(74) Vertreter: Müller-Boré & Partner Patentanwälte PartG mbB
(86) Internationale Anmeldenummer: PCT/DE2005/000747
(87) Internationale Veröffentlichungsnummer: WO 2005/113063

(56) Entgegenhaltungen:
- EP-A- 1 145 736
- EP-A2- 1 145 735
- DE-A1- 10 211 766
- US-A- 5 269 303
- US-A- 5 299 569
- TASS P A: "Desynchronizing double-pulse phase resetting and application to deep brain stimulation" BIOLOGICAL CYBERNETICS, SPRINGER VERLAG, BERLIN, DE, Bd. 85, Nr. 5, November 2001 (2001-11), Seiten 343-354, XP002245895 ISSN: 0340-1200
- P. A. TASS: "Development of Demand-Controlled Deep Brain Stimulation Techniques Based on Stochastic Phase Resetting" AIP CONFERENCE PROCEEDINGS, Bd. 665, Nr. 1, 28. Mai 2003 (2003-05-28), Seiten 242-249, XP002343164

## Beschreibung

Die Erfindung betrifft eine Vorrichtung zur Behandlung von Patienten mittels Hirnstimulation nach dem Oberbegriff des Anspruchs 1.

Bei Patienten mit neurologischen oder psychiatrischen Erkrankungen wie beispielsweise Morbus Parkinson, essentiellem Tremor, Dystonie oder Zwangserkrankungen sind Nervenzellenverbände in umschriebenen Bereichen des Gehirns, z.B. des Thalamus und der Basalganglien, krankhaft aktiv, zum Beispiel übersteigert synchron. In diesem Falle bildet eine große Anzahl von Neuronen synchron Aktionspotentiale aus, das heißt die beteiligten Neuronen feuern übermäßig synchron. Beim gesunden Patienten feuern die Neuronen in diesen Hirngebieten qualitativ anders, zum Beispiel auf unkorrelierte Weise.

Beim Morbus Parkinson verändert die pathologisch synchrone Aktivität die neuronale Aktivität in Arealen der Großhirnrinde, wie zum Beispiel im primären motorischen Cortex, indem sie diesen beispielsweise ihren Rhythmus aufzwingt, so daß schließlich die von diesen Arealen gesteuerten Muskeln pathologische Aktivität, z. B. ein rhythmisches Zittern, entfalten.

Bei Patienten, welche medikamentös nicht mehr behandelt werden können, wird, je nachdem, ob die Erkrankung ein- oder beidseitig auftritt, eine Tiefenelektrode implantiert. Unter der Haut führt dabei ein Kabel vom Kopf zum sogenannten Generator, welcher ein Steuergerät mit einer Batterie umfaßt, und beispielsweise im Bereich des Schlüsselbeins unter der Haut implantiert ist. Über die Tiefenelektroden wird eine Dauerreizung mit einer hochfrequenten periodischen Abfolge (mit einer Frequenz von > 100 Hz) von Einzelreizen, zum Beispiel Rechteckpulsen (pulse train) durchgeführt. Ziel dieser Methode ist es, das Feuern der Neuronen in den Zielgebieten zu unterdrücken. Diese Standard-Tiefenstimulation wirkt wie eine reversible Läsionierung - also wie eine reversible Ausschaltung des Gewebes. Die Wirkmechanismen, d. h. wie genau die Standard-Reizung funktioniert, ist noch nicht hinreichend geklärt.

Die bisher eingesetzte Methode hat jedoch einige Nachteile. So ist der bei der Dauerstimulation erreichte Energieverbrauch sehr hoch, so daß der Generator inklusive Batterie häufig schon nach ca. ein bis drei Jahren operativ ausgetauscht werden muß.

Besonders nachteilig ist jedoch, daß die hochfrequente Dauerstimulation als unphysiologischer also unnatürlicher Input im Bereich des Gehirns, zum Beispiel des Thalamus bzw. der Basalganglien im Laufe von wenigen Jahren zur Adaptation der betroffenen Nervenzellenverbände führen kann. Um denselben Stimulationserfolg zu erzielen, muß dann infolge dieser Anpassung mit höherer Reizamplitude stimuliert werden. Je größer die Reizamplitude ist, desto größer ist die Wahrscheinlichkeit, daß es infolge der Reizung von Nachbararealen zu Nebenwirkungen - wie Dysarthrie (Sprechstörungen), Dysäthesie (zum Teil sehr schmerzhafte Mißempfindungen), cerebellärer Ataxie (Unfähigkeit, ohne fremde Hilfe zu stehen), Depression oder Schizophrenie-artigen Symptomen etc - kommt. Diese Nebenwirkungen können vom Patienten nicht toleriert werden. Die Behandlung verliert daher in diesen Fällen nach wenigen Jahren ihre Wirksamkeit.
Aus dem Dokument "Desynchronization by Means of a Coordinated Reset of Neural Sub-Populations" von P. A. Tass, Progess of Theoretical Physics Supplement No. 150, 2003, Seiten 281 bis 296 ist ein Verfahren zur Desynchronisation einer Neuronenpopulation bekannt, bei dem zeitversetzte und einander überlappende phasenresettende Hochfrequenzpulszüge appliziert werden. Aus dem Dokument "Desynchronizing double-pulse phase resetting and application to deep brain stimulation" von P. A. Tass, Biol. Cybern. 85, 2001, Seiten 343 bis 354 ist ein Verfahren unter Verwendung eines ersten resettenden Pulses gefolgt von einem zweiten schwächeren Puls bekannt. Die Dokumente EP 1 145 735 A2 und US 5,269,303 offenbaren weitere Anwendungen von Neurostimulatoren.
Das Dokument DE 102 11 766 A1 offenbart eine Vorrichtung zur Behandlung von Patienten mittels Hirnstimulation, bei der zur Desynchronisation der neuronalen Aktivität bei Erkennen eines pathologischen Merkmals durch eine Steuerung entweder a) ein Hochfrequenzpulszug gefolgt von einem Einzelpuls oder b) ein Niederfrequenzpulszug gefolgt durch ein Einzelpuls oder c) ein Hochfrequenzpulszug appliziert werden.
Der Nachteil dieses in der DE 102 11 766 A1 beschriebenen Verfahrens ist, dass die Einzelpulse nicht immer optimal wirksam sind. Bei nicht ausreichender Wirksamkeit muss die Amplitude der Einzelreize relativ hoch gewählt werden, so dass es - z. B. durch Ausbreitung des Stimulationsstroms auf benachbarte Gehirngebiete - zum Auftreten von Nebenwirkungen kommen kann.

Das Konzept des Resets der neuronalen Aktivität einer Neuronenpopulation ist dem Fachmann wohlvertraut und beispielsweise in folgenden Dokumenten beschrieben: A. Jackson, R. L. Spinks, T. C. B. Freeman, D. M. Wolpert, R. N. Lemon: "Rhythm generation in monkey motor cortex explored using pyramidal tract stimulation", J. Physiol. 541.3, 685-699 (2002); A. T. Winfree: "Phase Control of Neural Pacemakers", Science 197, 761-763 (1977); J. Lewis, M. Bachoo, C. Polosa, L. Glass: "The effects of superior laryngeal nerve stimulation on the respiratory rhythm: phase-resetting and aftereffects", Brain Research 517, 44-50 (1989); S. Kitano, A. Komatsu: "Central respiratory oscillator: phase-response analysis", Brain Research 439, 19-30 (1988) und F. L. Eldridge, D. Paydarfar, P. G. Wagner, R. T. Dowell: "Phase resetting of respiratory rhythm: effect of changing respiratory 'drive'", Am. J. Physiol. 257 (Regulatory Integrative Comp. Physiol. 26), R271-R277 (1989).

Es ist daher die Aufgabe der Erfindung, eine Vorrichtung zu schaffen, die eine effizientere Behandlung als mit der Vorrichtung nach DE 102 11 766 A1 ermöglicht, bei der Symptome der jeweiligen Erkrankung vermindert oder vollständig beseitigt werden. Dabei soll die Aktivität der betroffenen Nervenzellenverbände nicht einfach nur unterdrückt werden, sondern sie soll dem gesunden Funktionszustand näher gebracht werden. Weiterhin sollen die Nebenwirkungen, wie beispielsweise die bereits oben genannte Dysarthrie, Dysäthesie, cerebelläre Ataxie, Depression- oder Schizophrenie-artige Symptome etc., die sich nach den Methoden gemäß dem Stand der Technik ergeben, beseitigt oder mindestens gemindert werden. Im Vergleich zu der Vorrichtung und dem Verfahren nach der DE 102 11 766 A1 soll ein Verfahren und eine Vorrichtung geschaffen werden, welche mit geringeren Reizamplituden auskommen insbesondere um Nebenwirkungen für den Patienten zu reduzieren oder auszuschalten.

Ausgehend vom Oberbegriff des Anspruchs 1 wird die Aufgabe erfindungsgemäß mit den im kennzeichnenden Teil des Anspruchs 1 angegebenen Merkmalen gelöst.

Mit der erfindungsgemäßen Vorrichtung ist es nunmehr möglich, Patienten zu behandeln, ohne daß eine Adaptation an den unphysiologischen Dauerreiz stattfindet, wobei die oben genannten Nebenwirkungen vermindert oder unterbunden werden. Durch die Verwendung der erfindungsgemäßen Vorrichtung kann zusätzlich der Batterie- bzw. Stromverbrauch drastisch reduziert werden, weswegen die Batterien weniger häufig ausgetauscht bzw. aufgeladen werden müssen. Die erfindungsgemäße Vorrichtung kann mit geringerer Reizamplitude arbeiten und führt zu einem verbesserten therapeutischen Effekt im Vergleich zur Vorrichtung aus der DE 102 11 766 A1.

Vorteilhafte Weiterbildungen der Erfindung sind in den Unteransprüchen angegeben.

Die Zeichnungen zeigen eine beispielhafte Ausführungsform der erfindungsgemäßen Vorrichtung sowie erfindungsgemäße Reizmuster.

Es zeigt:
- Fig.1:: Ein Blockschema der Vorrichtung
- Fig.2: Beispielhafte erfindungsgemäße Pulsfolgen

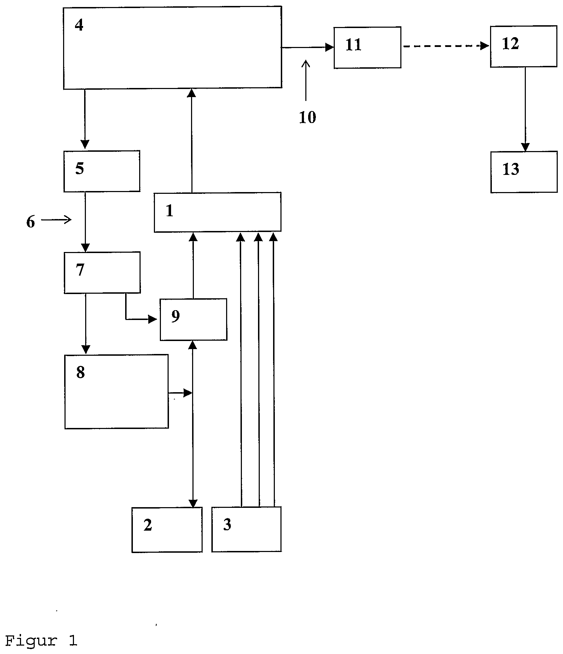

Die in Figur 1 dargestellte erfindungsgemäße Vorrichtung umfasst einen Trennverstärker (1), an den mindestens eine Elektrode (2) sowie Sensoren (3) zur Erfassung von physiologischen Messsignalen angeschlossen sind. Der Trennverstärker steht weiterhin mit einer Einheit (4) zur Signalverarbeitung und Steuerung in Verbindung, welche an einen optischen Sender für die Stimulation (5) angeschlossen ist. Der optische Sender (5) ist über Lichtwellenleiter (6) mit einem optischen Empfänger (7) verbunden, welcher mit einer Stimulatoreinheit (8) zur Signalerzeugung in Verbindung steht. Die Stimulatoreinheit (8) für die Signalerzeugung steht mit der Elektrode (2) in Verbindung. Am Eingangsbereich der Elektrode (2) in den Trennverstärker (1) befindet sich ein Relais (9) oder Transistor. Die Einheit (4) steht über eine Leitung (10) mit einem Telemetriesender (11) in Verbindung, welcher mit einem Telemetrieempfänger (12) in Verbindung steht, der sich außerhalb des zu implantierenden Geräts befindet und an den ein Mittel zur Visualisierung, Verarbeitung und Speicherung der Daten (13) angeschlossen ist.

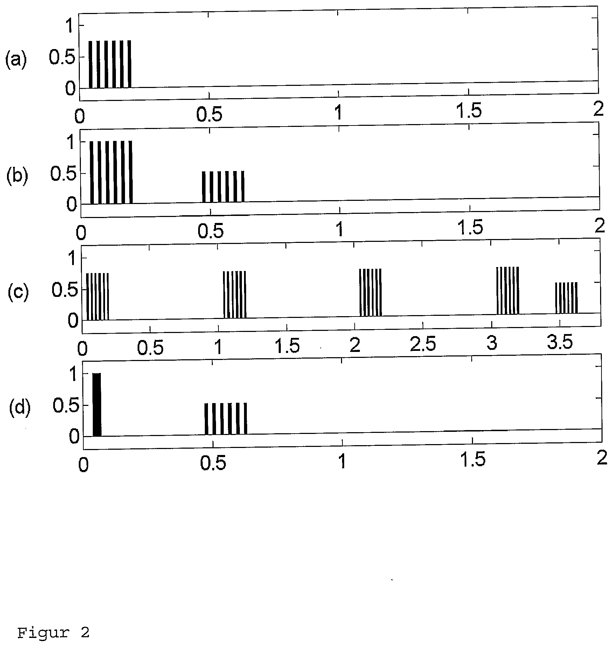

Figur 2 zeigt beispielhaft die erfindungsgemäßen Reizmuster. In den Figuren 2a bis 2d entspricht die Ordinate der Stromstärke und die Abszisse der Zeit, wobei beide in willkürlichen Einheiten dargestellt sind. Ein einzelner Einzelpuls ist in allen Figuren schematisch als Rechteckblock dargestellt.

In Figur 2a ist ein einzelner Hochfrequenzpulszug, der aus 6 Einzelpulsen besteht, dargestellt.

In Figur 2b ist ein resettender Hochfrequenzpulszug, der von einem desynchronisierenden Hochfrequenzpulszug gefolgt wird, dargestellt.

In Figur 2c ist eine niederfrequente resettende Abfolge von Hochfrequenzpulszügen, die von einem desynchronisierenden Hochfrequenzpulszug gefolgt wird, dargestellt.

In Figur 2d ist ein resettender Einzelpuls, der von einem desynchronisierenden Hochfrequenzpulszug gefolgt wird, dargestellt.

Als Sensoren (3) können beispielsweise epikortikale Elektroden, Tiefenelektroden, Hirnelektroden oder periphere Elektroden eingesetzt werden.

Bei der Elektrode (2) handelt es sich um mindestens zwei Drähte, an deren Enden eine Potentialdifferenz zum Zwecke der Stimulation angelegt wird. Die Elektrode (2) ist ein Mittel zur Reizapplikation. Im weiteren Sinne kann sie auch ein Mittel zur Messung von physiologischen Signalen sein. Es kann sich dabei um Makro- oder Mikroelektroden handeln. Zusätzlich aber nicht zwingend kann über die Elektrode (2) eine Potentialdifferenz gemessen werden, um eine pathologische Aktivität festzustellen. In einer weiteren Ausführungsform kann die Elektrode (2) auch nur aus einem einzelnen Draht bestehen. In diesem Falle wird zum Zwecke der Stimulation eine Potentialdifferenz zwischen dem Ende dieses Drahtes einerseits und einem metallischen Gegenstück andererseits angelegt. Bei dem metallischen Gegenstück kann es sich beispielsweise um ein Gehäuse der Vorrichtung oder eines Teils davon oder eine andere beliebige Elektrode oder einen anderen metallischen Gegenstand handeln, der analog wie der Draht der Elektrode (2) mit der Stimulatoreinheit (8) verbunden ist. In einer weiteren Ausführungsform kann Elektrode (2) auch aus mehr als zwei einzelnen Drähten bestehen, die sowohl für die Ermittlung eines Messsignals im Gehirn als auch für die Stimulation herangezogen werden können. Beispielsweise können vier Drähte in einem Leiterkabel untergebracht sein, wobei zwischen verschiedenen Enden eine Potenzialdifferenz angelegt oder gemessen werden kann. Hierdurch lässt sich die Größe des abgeleiteten bzw. stimulierten Zielgebietes variieren. Die Anzahl der Drähte, aus welchen sich die Elektrode aufbaut, ist nach oberen Werten hin lediglich durch die damit verbundene Dicke des in das Gehirn einzuführenden Kabels begrenzt, so dass möglichst wenig Hirnmaterial beschädigt werden soll. Handelsübliche Elektroden umfassen vier Drähte, es können jedoch auch fünf, sechs oder mehr Drähte, aber auch nur drei Drähte umfasst sein. Die geeigneten Elektroden sind dem Fachmann bekannt und nicht auf die beispielhaft aufgeführten Elektroden beschränkt.

Für den Fall, daß die Elektrode (2) mehr als zwei Drähte umfaßt, können mindestens zwei dieser Drähte auch als Sensor (3) fungieren, so dass in diesem Spezialfall eine Ausführungsform vorliegt, bei der die Elektrode (2) und der Sensor (3) in einem einzigen Bauteil vereint sind. Die Drähte der Elektrode (2) können unterschiedliche Längen haben, so daß sie in verschiedene Hirntiefen eindringen können. Besteht die Elektrode (2) aus n Drähten, so kann eine Stimulation über mindestens ein Paar von Drähten erfolgen, wobei bei der Paarbildung jede Unterkombination von Drähten möglich ist. Neben diesem Bauteil können zusätzlich nicht mit Elektrode (2) baulich vereinte Sensoren (3) vorhanden sein.

Die Einheit zur Signalverarbeitung und Steuerung 4 umfasst Mittel für eine univariate und/oder bivariate Datenverarbeitung, wie sie beispielsweise in "Detection of n:m Phase Locking from Noisy Data: Application to Magnetoencephalography" von P. Tass, et.al. in Physical Review Letters, 81,3291 (1998) beschrieben ist.

Erfindungsgemäß ist die Vorrichtung mit Mitteln ausgestattet, welche die Signale der Elektrode (2) und oder der Sensoren (3) als pathologisch erkennen und im Falle des Vorliegens eines pathologischen Musters über die Elektrode (2) Reize abgeben, die bewirken dass die pathologische neuronale Aktivität entweder kurzfristig unterdrückt oder so modifiziert wird, daß sie der natürlichen, physiologischen Aktivität näher kommt. Die pathologische Aktivität unterscheidet sich von der gesunden Aktivität durch eine charakteristische Veränderung ihres Musters und/oder ihrer Amplitude, die dem Fachmann bekannt sind und die mit bekannten Methoden detektiert werden kann.

Die Mittel zum Erkennen des pathologischen Musters sind dabei ein Rechner, der die gemessenen Signale der Elektrode (2) und/oder des Sensors (3) verarbeitet und mit im Rechner gespeicherten Daten vergleicht. Der Rechner verfügt über einen Datenträger, welcher Daten speichert, die im Rahmen einer Eichprozedur ermittelt wurden. Beispielhaft können diese Daten ermittelt werden, indem in einer Serie von Testreizen die Stimulationsparameter systematisch variiert werden und der Erfolg der Stimulation über die Elektrode (2) und/oder den Sensor (3) mittels der Steuereinheit (4) ermittelt wird. Die Ermittlung kann durch uni- und/oder bi- und/oder multivariate Datenanalyse zur Kennzeichnung der Frequenzeigenschaften und der Interaktion (z. B. Kohärenz, Phasensynchronisation, Direktionalität und Reiz-Antwort-Beziehung) erfolgen, wie sie beispielsweise in P.A. Tass: "Phase resetting in Medicine and Biology. Stochastic Modelling and Data Analysis." Springer Verlag, Berlin 1999 offenbart ist.

Die erfindungsgemäße Vorrichtung umfasst daher einen Rechner, welcher einen Datenträger beinhaltet, der die Daten des Krankheitsbildes trägt, mit den Meßdaten vergleicht und im Falle des Auftretens pathologischer Aktivität ein Reizsignal an die Elektrode (2) abgibt, so daß eine Stimulation des Hirngewebes erfolgt. Die in dem Datenträger gespeicherten Daten des Krankheitsbildes können entweder personenspezifische, durch Eichung bestimmte optimal Stimulationsparameter sein oder ein Datenmuster, welches aus einem Patientenkollektiv bestimmt worden ist und typischerweise auftretende optimale Stimulationsparameter repräsentiert. Der Rechner erkennt das pathologische Muster und/oder die pathologische Amplitude.

Die Steuereinheit (4) kann beispielsweise einen Chip oder eine andere elektronische Vorrichtung mit vergleichbarer Rechenleistung umfassen.

Die Steuereinheit (4) steuert die Elektrode (2) vorzugsweise in folgender Weise an. Die Steuerdaten werden von der Steuereinheit (4) an einen optischen Sender für die Stimulation (5) weitergegeben, welcher über den Lichtleiter (6) den optischen Empfänger (7) ansteuert. Durch das optische Einkoppeln von Steuersignalen in den optischen Empfänger (7), wird eine galvanische Entkopplung der Stimulationssteuerung von der Elektrode (2) bewirkt. Dies bedeutet, dass eine Einstreuung von Störsignalen von der Einheit zur Signalverarbeitung und Steuerung (4) in die
Elektrode (2) verhindert wird. Als optischer Empfänger (7) kommt beispielsweise eine Photozelle in Betracht. Der optische Empfänger (7) gibt die über den optischen Sender für die Stimulation (5) eingegebenen Signale an die Stimulatoreinheit (8) weiter. Über die Stimulatoreinheit (8) werden dann gezielte Stimuli über die Elektroden (2) an die Zielregion im Gehirn weitergegeben. Für den Fall, dass über die Elektrode (2) auch gemessen wird, wird ausgehend vom optischen Sender für die Stimulation (5) über den optischen Empfänger (7) auch ein Relais (9) angesteuert, was die Einstreuung von Störsignalen verhindert. Das Relais (9) oder der Transistor stellt sicher, dass die neuronale Aktivität unmittelbar nach jedem Stimulus wieder gemessen werden kann, ohne dass der Trennverstärker übersteuert. Die galvanische Entkopplung muß nicht zwingend durch eine optische Einkopplung der Steuersignale erfolgen, vielmehr können auch andere alternative Steuerungen verwendet werden. Diese können beispielsweise akustische Einkopplungen zum Beispiel im Ultraschallbereich sein. Eine störungsfreie Steuerung kann auch beispielsweise unter Zuhilfenahme geeigneter analoger oder digitaler Filter realisiert werden.

Weiterhin steht die erfindungsgemäße Vorrichtung vorzugsweise mit Mitteln zur Visualisierung und Verarbeitung der Signale sowie zur Datensicherung (13) über den Telemetriempfänger (12) in Verbindung. Dabei kann die Einheit (13) über die oben erwähnten Verfahren zur uni- und/oder bi- und/oder multivariaten Datenanalyse verfügen.

Weiterhin kann die erfindungsgemäße Vorrichtung über den Telemetriempfänger (13) mit einer zusätzlichen Referenzdatenbank in Verbindung stehen, um beispielsweise den Eichprozess zu beschleunigen.

In der Neurochirurgie werden typischerweise zwei Stimulationsarten verwendet: 1. Hochfrequenz-Dauerstimulation (zur Unterdrückung neuronaler Aktivität) und 2. Niederfrequenz-Stimulation (zur Verstärkung bzw. Anregung neuronaler Aktivität). Die Frequenz der Hochfrequenz-Dauerstimulation ist typischerweise größer als 100 Hz, z.B. 130 Hz. Die Frequenz der Niederfrequenz-Dauerstimulation liegt hingegen bei Werten um 2 Hz bis 30 Hz.

Im Gegensatz hierzu werden in der erfindungsgemäßen Vorrichtung neuartige Reizformen verwendet, die besonders effizient die Phasendynamik und das Ausmaß der Synchronisation neuronaler rhythmischer Aktivität beeinflussen. Es hat sich überraschenderweise gezeigt, dass die unten beschriebenen, aus kurzen Hochfrequenz-Pulszügen zusammengesetzten komplexeren Reizabfolgen auf besonders wirksame Weise die krankhaft synchrone Aktivität der natürlichen, nicht krankhaften Aktivität nahebringen oder ihr vollkommen angleichen.

Mit der erfindungsgemäßen Vorrichtung wird die pathologische neuronale Aktivität über eine Elektrode (2), wie eine a) Hirnelektrode, z. B. eine Tiefenelektrode, eine b) epikortokale Elektrode oder über c) eine Muskelelektrode gemessen und dient als Feedback-Signal, also Steuerungssignal, für eine bedarfsgesteuerte Stimulation. Das Feedback-Signal aus dem Sensor (3) wird über eine Leitung an den Trennverstärker (1) übermittelt. Alternativ kann das Feedback-Signal auch - ohne Verwendung eines Trennverstärkers - telemetrisch übertragen werden. Im Falle der telemetrischen Übertragung ist Sensor (3) mit einem Verstärker über ein Kabel verbunden. Der Verstärker ist mit einem Telemetriesender über ein Kabel verbunden. In diesem Fall sind Sensor (3) und Verstärker und Telemetriesender zum Beispiel im Bereich einer betroffenen Extremität implantiert, während der Telemetrieempfänger über ein Kabel mit der Steuereinheit (4) verbunden ist. Das bedeutet, dass die Aktivität gemessen und das Messsignal als Auslöser für eine bedarfsgesteuerte Stimulation eingesetzt wird.

Für die Messung der neuronalen Aktivität, gibt es folgende verschiedene Möglichkeiten:
I. Messung über die Hirnelektrode a) (Elektrode (2)), die in diesem Fall die Funktion eines Sensors (3) mit übernehmen), über die auch stimuliert wird. Wenn Elektrode (2) aus mehr als drei Drähten besteht, können mindestens zwei dieser Drähte als Sensor (3) fungieren, wobei in diesem Fall über diese Drähte nicht stimuliert wird.
II. Messung der neuronalen Aktivität aus tieferen Bereichen des Gehirns, wie Thalamus oder Basalganglien über die Tiefenelektrode a') (Sensor (3)), über die nicht stimuliert wird. In diesem Fall wird neben der als Elektrode (2) fungierenden Tiefenelektrode a) eine weitere Tiefenelektrode a') als Sensor (3) verwendet.
III. Messung von neuronaler Aktivität, die aus der Hirnrinde stammt, entweder über eine implantierte Elektrode b) oder vorzugsweise eine atraumatische epikortikale Elektrode b) (Sensor (3)), d. h. eine Elektrode die auf dem Gehirn aufliegt und fixiert ist, aber nicht in das Gewebe eindringt und auf diese Weise ein lokales Elektroencephalogramm von einem betroffenen Areal der Hirnrinde, z. B. dem primären motorischen Cortex, ableitet.
IV. Bei Patienten, die primär an einem Tremor leiden, kann auch die Messung von muskulärer Aktivität durch Elektroden c) (Sensor (3), vorzugsweise telemetrisch mit Steuereinheit (4) verbunden) im Bereich der betroffenen Muskulatur erfolgen.

Die pathologische neuronale Aktivität kann prinzipiell auch in unterschiedlichen Neuronenpopulationen auftreten. Deswegen können auch mehrere, über Elektrode (2) und/oder Sensoren (3) gemessene Signale zur Steuerung der Stimulation verwendet werden. Immer wenn in mindestens einer der Neuronenpolulationen ein pathologisches Merkmal der Aktivität detektiert wird, wird eine Reizung ausgelöst.
Die Elektrode (2) kann auch die Funktion eines Sensors (3) übernehmen. Dies ermöglicht, die Aktivität der Neuronenpopulation am Behandlungspunkt der Elektrode (2) abzuleiten.

Das Messsignal oder die Messsignale dient oder dienen als Feedback-Signale. Das bedeutet eine Stimulation erfolgt in Abhängigkeit der über das Messsignal erfaßten Aktivität. Immer, wenn ein pathologisches Merkmal der neuronalen Aktivität (das heißt, pathologisch gesteigerte Amplitude oder pathologisch gesteigert ausgeprägtes Aktivitätsmuster) auftritt und/oder sich steigert, wird stimuliert.
Eine Reizung erfolgt also erfindungsgemäß dann, wenn pathologisch synchronisierte Nervenzell-Tätigkeit im Zielareal (abgeleitet über Elektrode (2)) (z. B. bei der Parkinsonschen Erkrankung in Bereichen des Thalamus) oder in einem anderen für die Erkrankung relevanten Areal oder Muskel (über Sensoren (3) abgeleitet) vorliegt. Dies wird beispielsweise dadurch festgestellt, dass die über Elektrode (2) und/oder Sensoren (3) gemessenen Signale in dem Frequenzbereich bandpassgefiltert werden, der für die pathologische Aktivität charakteristisch ist. Sobald ein bandpassgefiltertes Meßsignal einen - im Rahmen der Eichprozedur bestimmten - Schwellenwert überschreitet, wird über Steuereinheit (4) der nächste Steuerimpuls an den optischen Sender (5) weitergeleitet, der über den Lichtwellenleiter (6) und den optischen Empfänger (7) die über Elektrode (2) erzeugten Reize hervorruft. Ziel ist es hierbei nicht, wie bei der Standard-Dauerstimulation das Feuern der Neuronen einfach zu unterdrücken. Vielmehr soll bedarfsgerecht nur die krankhaft gesteigerte Synchronisation der Nerverzellen behoben werden. Das heißt, die Nervenzellverbände im Zielareal werden desynchronisiert, wobei sie weiterhin aktiv sind, also Aktionspotentiale ausbilden. Damit sollen die betroffenen Nervenzellen näher an ihren physiologischen - also unkorreliert feuernden - Zustand gebracht werden, anstatt daß einfach nur ihre Aktivität komplett unterdrückt wird. Hierfür können mehrere verschiedene desynchronisierende Verfahren, die auf dem auf dem Prinzip des "stochastischen Phase resetting" basieren, verwendet werden. Hierbei wird ausgenutzt, dass eine synchronisierte Neuronenpopulation durch Applikation eines elektrischen Reizes der richtigen Intensität und Dauer desynchronisiert werden kann, vorausgesetzt, der Reiz wird in einer vulnerablen Phasenlage der krankhaften rhythmischen Aktivität verabreicht. Diese optimalen Stimulationsparameter (Intensität, Dauer und vulnerable Phase) werden im Rahmen der Eichprozedur beispielsweise durch systematische Variation dieser Parameter und Vergleich mit dem Stimulationserfolg (z. B. Dämpfung der Amplitude des bandpassgefilterten Feedback-Signals) ermittelt. Im Falle der Verwendung der Telemetrievorrichtung 11-13 kann die Eichung durch Verwendung sogenannter Phase resetting-Kurven beschleunigt werden. Die Stimulation mit einzelnem Hochfrequenzpulszug ist nur effizient, wenn der Reiz bei der oder nahe genug bei der vulnerablen Phase der zu stimulierenden Aktivität appliziert wird. Alternativ können auch komplexe Stimulationsformen verwendet werden. Diese setzen sich aus einem resettenden (die Dynamik der zu stimulierenden Neuronenpopulation kontrollierenden, zum Beispiel neu startenden) Stimulus und einem desynchronisierenden Hochfrequenzpulszug zusammen. Ein resettender Reiz ist zum Beispiel ein kurzer Hochfrequenzpulszug. Der Vorteil dieser komplexeren Verfahren ist, daß die komplexen Stimulationsformen unabhängig vom dynamischen Zustand der zu stimulierenden Neuronenpopulation eine Desynchronisation hervorrufen.

Im Falle der Verwendung einzelnen kurzen Hochfrequenzpulszugs muß die Steuereinheit (4) bei Überschreiten des durch die Eichung ermittelten Schwellenwerts mittels durch die Elektronik (Steuereinheit (4)) realisierten Standard-Prädiktionsalgorithmen das zeitliche Auftreten der vulnerablen Phase vorausberechnen, um diese präzise genug zu treffen. Bei der Applikation der erfindungsgemäßen komplexen Reize muß Steuereinheit (4) bei Überschreiten des durch die Eichung ermittelten Schwellenwerts lediglich einen neuen komplexen Reiz derselben Art hervorrufen.

Im Folgenden soll die Funktionsweise der erfindungsgemäßen Vorrichtung sowie das Behandlungsverfahren erläutert werden. Erfindungsgemäß kann mindestens eine Komponente aus der Gruppe der Reizmuster a) bis d) der einfachen Reize und/oder komplexen Reize verwendet werden:
a) Stimulation mit einem kurzen Hochfrequenzpulszug.
b) Stimulation mit einem resettenden, kurzen Hochfrequenzpulszug, gefolgt von einem desynchronisierenden kurzen Hochfrequenzpulszug,
c) Stimulation mit einer resettenden niederfrequenten Abfolge von kurzen Hochfrequenzpulszügen, gefolgt von einem desynchronisierenden Hochfrequenzpulszug.
d) Stimulation mit einem resettenden, Einzelpuls gefolgt von einem desynchronisierenden kurzen Hochfrequenzpulszug. Dabei handelt es sich beim Reizmuster a) um einen einfachen Reiz und bei den Reizmustern c)-d) um komplexe Reize

Unter einem kurzen Hochfrequenzpulszug im Sinne der Erfindung wird eine kurze hochfrequente Abfolge von elektrischen Einzelreizen verstanden.

Kurz bedeutet, dass diese Abfolge aus mindestens zwei, vorzugsweise 3,4,5,6,7,8,9,10,11,12,13,14,15,16,17,18,19,20,50 oder bis zu 100 Einzelreizen besteht.
Vorzugsweise haben alle Hochfrequenzpulszüge die gleiche Anzahl von Einzelreizen. Es können aber auch mindestens zwei Hochfrequenzpulszüge aus einer unterschiedlichen Anzahl von Einzelreizen bestehen.
Die Anzahl der Einzelreize, aus denen ein resettender Hochfrequenzpulszug besteht, liegt im Bereich von zwei, vorzugsweise 3,4,5,6,7,8,9,10,11,12,13,14,15,16,17,18,19, 20, 50 oder bis zu 100 Einzelreizen.
Vorzugsweise liegt die Anzahl der Einzelreize, aus denen ein resettender Hochfrequenzpulszug besteht, im Bereich von 4 bis 20 Einzelreizen.
Die Anzahl der Einzelreize, aus denen ein desynchronisierender Hochfrequenzpulszug besteht, liegt im Bereich von zwei, vorzugsweise 3,4,5,6,7,8,9,10,11,12,13,14,15,16,17,18,19,20, 50 oder bis zu 100 Einzelreizen. Vorzugsweise liegt die Anzahl der Einzelreize, aus denen ein desynchronisierender Hochfrequenzpulszug besteht, im Bereich von 3 bis 15 Einzelreizen.
Hochfrequent bedeutet im Sinne der Erfindung, dass die Frequenz vorzugsweise zwischen 50 bis 250 Hz, vorzugsweise zwischen 80 und 150 Hz besonders bevorzugt zwischen 100 und 140 liegt.

Vorzugsweise haben alle Hochfrequenzpulszüge die gleiche Frequenz. Es können aber auch mindestens zwei Hochfrequenzpulszüge aus Einzelreizen unterschiedlicher Frequenz bestehen.
Die zeitliche Dauer eines kurzen Hochfrequenzpulszugs findet dadurch eine natürliche Grenze, dass der kurze Hochfrequenzpulszug vorzugsweise die Periodenlänge der krankhaften neuronalen Oszillation nicht überschreiten soll, um wirksam zu sein. Insofern sind die angegebenen Werte nicht beschränkend.

Unter einem elektrischen Einzelreiz wird ein dem Fachmann bekannter, im wesentlichen ladungsneutraler elektrischer Reiz verstanden.
Ladungsneutral im Sinne der Erfindung bedeutet, dass das Zeitintegral des Ladungseintrags im wesentlichen null ist.
Der zeitliche Verlauf des Ladungseintrages kann dabei symmetrisch oder unsymmetrisch sein. Das heißt, bei diesen biphasischen Einzelpulsen können der kathodische und anodische Teil des Einzelpulses symmetrisch oder unsymmetrisch sein. Im symmetrischen Fall sind dabei der kathodische und der anodische Teil des Einzelpulses bis auf das Vorzeichen des Stromflusses identisch.
Die Amplitude der Hochfrequenzpulszüge kann in einer Größenordnung von 0 bis 16 V liegen. Vorzugsweise liegt die Amplitude der Hochfrequenzpulszüge zwischen 2 und 7 V. Der übliche Widerstand von Elektrode und Hirngewebe liegt beispielsweise im Bereich von 800 bis 1200 Ω.
Vorzugsweise ist die Amplitude für alle Hochfrequenzpulszüge gleich, sie kann jedoch auch für mindestens zwei Hochfrequenzpulszüge unterschiedlich sein.
Die resettenden Hochfrequenzpulszüge sind im Vergleich zu den desynchronisiernden Hochfrequenzpulszügen vorzugsweise stärker. Das bedeutet, dass bei den resettenden Hochfrequenzpulszügen die Amplitude und/oder die Anzahl der Einzelpulse größer ist als bei einem desynchronisiernden Hochfrequenzpulszug.
Die Amplitude der Einzelreize, aus denen ein resettender Hochfrequenzpulszug besteht, liegt im Bereich von 0 bis 16 V, vorzugsweise zwischen 3 und 7 V.
Die Amplitude der Einzelreize, aus denen ein desynchronisierender Hochfrequenzpulszug besteht, liegt im Bereich von 0 bis 15 V, vorzugsweise zwischen 2 und 6 V.
Ein Hochfrequenzpulszug kann aus Einzelreizen bestehen, die vorzugsweise dieselbe Amplitude und/oder dieselbe Dauer haben. Es können jedoch auch mindestens zwei Einzelreize dieselbe Amplitude und/oder dieselbe Dauer haben.
Ein Hochfrequenzpulszug kann auch aus Einzelreizen bestehen, von denen mindestens zwei Einzelreize unterschiedliche Amplitude und/oder unterschiedliche Dauer haben. Die Dauer und/oder die Amplitude der Einzelreize kann durch deterministische und/oder stochastische Gesetzmäßigkeiten und/oder Kombinationen von beiden gegeben sein. Bei einer Kombination von stochastischen und deterministischen Gesetzmäßigkeiten handelt es sich um einen funktionellen Zusammenhang, bei dem deterministische und stochastische Terme funktionell, z. B. durch Addition oder Multiplikation miteinander verbunden sind. Zum Beispiel kann die Amplitude des j-ten Einzelpulses durch f(j) gegeben sein, wobei f eine deterministische Funktion und/oder ein stochastischer Prozess und/oder eine Kombination von beiden ist.
Analog wird im Folgenden unter einer Kombination deterministischen und stochastischen Gesetzmäßigkeiten ein funktionaler Zusammenhang verstanden, bei dem deterministische und stochastische Terme funktionell, z.B. durch Addition und/oder Multiplikation miteinander verbunden sind.
Eine niederfrequente Abfolge von kurzen Hochfrequenzpulszügen umfasst vorzugsweise 2-30, besonders bevorzugt 2-20 oder 2-10 Hochfrequenzpulszüge.

Die niederfrequente Abfolge von kurzen Hochfrequenzpulszügen besteht vorzugsweise aus einer periodischen Abfolge von kurzen Hochfrequenzpulszügen, deren Frequenz im wesentlichen der pathologischen Frequenz - beispielsweise beim Morbus Parkinson ca. 5 Hz - entspricht.
Eine niederfrequente Abfolge von kurzen Hochfrequenzpulszügen besteht vorzugsweise aus denselben Hochfrequenzpulszügen. Die Hochfrequenzpulszüge einer solchen niederfrequenten Abfolge können sich aber auch bezüglich ihres Musters unterscheiden.
Das Muster eines Hochfrequenzpulszugs umfasst folgende Eigenschaften:
A) die Anzahl der Einzelpulse,
B) die Dauern der einzelnen Einzelpulse,
C) die Pausen zwischen den einzelnen Einzelpulsen,
D) die Amplituden der einzelnen Einzelpulse.
Innerhalb einer niederfrequenten Abfolge von kurzen resettenden Hochfrequenzpulszügen kann das Muster von Hochfrequenzpulszug zu Hochfrequenzpulszug deterministisch und/oder stochastisch und/oder kombiniert deterministisch/stochastisch variiert werden. Insbesondere kann innerhalb einer niederfrequenten Abfolge von kurzen Hochfrequenzpulszügen die Frequenz im einzelnen Hochfrequenzpulszug variiert werden.
Vorzugsweise wird bei mehrmaliger Applikation eines einfachen Reizes und/oder eines komplexen Reizes das Muster der jeweiligen Hochfrequenzpulszüge nicht variiert.
Es kann aber auch bei mehrmaliger Applikation eines Einfachen Reizes oder eines komplexen Reizes das Muster eines Hochfrequenzpulszugs von Applikation zu Applikation variiert werden. Es kann zum Beispiel bei einem Hochfrequenzpulszug in einem einfachen und/oder komplexen Reiz die Anzahl der Einzelreize und/oder deren Amplituden und/oder deren Dauern und/oder deren Pausen von Applikation zu Applikation deterministisch und/oder stochastisch und/oder kombiniert deterministisch/stochastisch variiert werden.

Bei mehrmaliger Applikation eines kurzen desynchronisierenden Hochfrequenzpulszugs kann somit dessen Muster von Applikation zu Applikation deterministisch und/oder stochastisch und/oder kombiniert deterministisch/stochastisch variiert werden. Insbesondere kann hierbei die Frequenz des desynchronisierenden Hochfrequenzpulszugs von Applikation zu Applikation variiert werden.
Ebenso kann bei mehrmaliger Applikation eines kurzen resettenden Hochfrequenzpulszugs dessen Muster von Applikation zu Applikation deterministisch und/oder stochastisch und/oder kombiniert deterministisch/stochastisch variiert werden. Insbesondere kann hierbei die Frequenz des desynchronisierenden Hochfrequenzpulszugs von Applikation zu Applikation variiert werden.
Wird ein kurzer Hochfrequenzpulszug zur Desynchronisation verwandt - wie unter a) bis d) beschrieben - so ist dessen Intensität, z. B. im Sinne des pro Zeiteinheit erfolgenden Ladungseintrages vorzugsweise geringer oder kleiner als die Intensität eines kurzen Hochfrequenzpulszugs, der zum Reset verwendet wird.
Die erfindungsgemäße Vorrichtung kann bei mehrmaliger, bedarfsgesteuerter Applikation gemäß stochastischen und/oder deterministischen und/oder kombiniert stochastisch-deterministischen Gesetzmäßigkeiten zwischen den unter a)-d) beschriebenen Reizformen wählen.

In einer bevorzugten Ausführungsform ist die Vorrichtung mit Mitteln zur kabellosen Übertragung von Daten, wie beispielsweise der Meßsignale und Stimulations-Steuer-Signale ausgestattet, damit eine Datenübertragung vom Patienten zu einem externen Empfänger zum Beispiel zum Zweck der Therapieüberwachung und -optimierung stattfinden kann. Auf diese Weise kann frühzeitig erkannt werden, ob die verwendeten Stimulationsparameter nicht mehr optimal sind. Zusätzlich kann durch eine kabellose Übertragung von Daten auf eine Referenz-Datenbank zurückgegriffen werden und frühzeitig auf typische Veränderungen der Reizbarkeit im Zielgewebe reagiert werden.
Erfindungsgemäß wird ein elektronisches Bauteil zu Verfügung gestellt, welches das Auftreten und den Wegfall eines pathologischen Merkmals des elektrischen Signals, welches von dem Sensor (3, 2) gemessen wird, erkennt und bei Eintreten des pathologischen Merkmals mindestens eine Pulsfolge aus der Gruppe nach dem Muster a) bis d) auf die Elektrode (2) abgibt und das Reizmuster bei Wegfall des pathologischen Merkmals abschaltet. Es umfaßt in einer bevorzugten Ausführungsform eine univariate Datenverarbeitung und/oder weiterhin eine multivariate und/oder bivariate Datenverarbeitung. Vorzugsweise ist das elektronische Bauteil so ausgestaltet, daß mindestens eine der univariaten, bivariaten und multivariaten Datenverarbeitung mit Methoden der statistischen Physik arbeitet wobei die Methode der statistischen Physik aus dem Bereich des stochastischen Phase resetting stammen kann.

Die erfindungsgemäße Vorrichtung und das erfindungsgemäße elektronische Bauteil können in der Medizin, vorzugsweise in der Neurologie und Psychiatrie verwendet werden.

Es können beispielsweise folgende Krankheiten behandelt werden:
Morbus Parkinson, Parkinson Syndrom, Epilepsie, Dystonie, Zwangserkrankungen Alzheimer, Depression, essentieller Tremor, Tremor bei Multipler Sklerose, Tremor in Folge eines Schlaganfalls oder einer anderen tumorösen Gewebsschädigung.

Hierzu können folgende Hirnregionen stimuliert werden:
Bei:
Morbus Parkinson: Nucleus subthalamicus, Thalamus, Globus pallidum, Nucleus ventralis intermedius thalami.
Parkinson Syndrom: Nucleus Subthalamicus, Thalamus, Globus pallidum, Nucleus ventralis intermedius thalami.
Epilepsie: Fokale Herde, Hippocampus, Nucleus subthalamicus, Kleinhirn, thalamische Kerngebiete, Nucleus caudatus.
Dystonie: Globus pallidum
Zwangserkrankungen: Nucleus accumbens, capsula interna
Essentieller Tremor: Thalamus, Nucleus ventralis intermedius thalami
Alzheimer: Hippocampus
Depression: Hippocampus, Globus pallidum.
Tremor bei Multipler Skerose: Nucleus ventralis intermedius thalami.

## Patentansprüche

1. Vorrichtung zur Behandlung von Patienten, umfassend Mittel zum Stimulieren von Hirnregionen,
wobei die Vorrichtung folgende Komponenten umfasst:
- mindestens eine Elektrode (2) zur Stimulation einer Hirnregion mit einer krankhaft synchronen neuronalen Aktivität,
- mindestens einen Sensor (3, 2) zur Messung eines elektrischen Signals, und
- ein Steuermittel (4), welches das Auftreten eines pathologischen Merkmals des elektrischen Signals, welches von dem Sensor (3, 2) gemessen wurde, erkennt und bei Eintreten des pathologischen Merkmals eine Reizfolge auf die Elektrode (2) abgibt,
**dadurch gekennzeichnet,**
- **dass** die Reizfolge eine niederfrequente Abfolge von kurzen resettenden Hochfrequenzpulszügen gefolgt von einem kurzen desynchronisierenden Hochfrequenzpulszug umfasst, wobei aufeinanderfolgende resettende Hochfrequenzpulszüge einander stets nicht überlappen und die resettenden Hochfrequenzpulszüge jeweils eine größere Amplitude als der desynchronisierende Hochfrequenzpulszug aufweisen.

2. Vorrichtung nach Anspruch 1, wobei die resettenden Hochfrequenzpulszüge jeweils eine Amplitude zwischen 3 und 7 V aufweisen und der desynchronisierende Hochfrequenzpulszug eine Amplitude zwischen 2 und 6 V aufweist.

3. Vorrichtung nach Anspruch 1, wobei die resettenden Hochfrequenzpulszüge jeweils eine höhere Anzahl von Einzelpulsen als der desynchronisierende Hochfrequenzpulszug aufweisen.

4. Vorrichtung nach Anspruch 3, wobei die resettenden Hochfrequenzpulszüge jeweils aus 4 bis 20 Einzelpulsen bestehen und der desynchronisierende Hochfrequenzpulszug aus 3 bis 15 Einzelpulsen besteht.

## Claims

1. A device for treating patients, comprising means for stimulating brain regions, the device comprising the following components:
- at least one electrode (2) for stimulating a brain region having an abnormal synchronous neuronal activity,
- at least one sensor (3, 2) for measuring an electrical signal, and
- a control means (4) that detects the occurrence of a pathological feature of the electrical signal measured by the sensor (3, 2) and that emits a sequence of stimuli to the electrode (2) upon occurrence of the pathological feature,
**characterized in that**
- the sequence of stimuli comprises a low-frequency sequence of short resetting high-frequency pulse trains followed by a short desynchronizing high-frequency pulse train, wherein successive resetting high-frequency pulse trains always do not overlap one another and the resetting high-frequency pulse trains each have a greater amplitude than the desynchronizing high-frequency pulse train.

2. The device according to claim 1, wherein the resetting high-frequency pulse trains each have an amplitude between 3 and 7 V and the desynchronizing high-frequency pulse train has an amplitude between 2 and 6 V.

3. The device according to claim 1, wherein the resetting high-frequency pulse trains each have a higher number of single pulses than the desynchronizing high-frequency pulse train.

4. The device according to claim 3, wherein the resetting high-frequency pulse trains each consist of 4 to 20 individual pulses and the desynchronizing high-frequency pulse train consists of 3 to 15 individual pulses.

## Revendications

1. Dispositif pour le traitement de patients, comprenant des moyens pour stimuler des régions du cerveau,
sachant que le dispositif comprend les composants suivants :
- au moins une électrode (2) pour la stimulation d'une région du cerveau avec une activité neuronale pathologiquement synchrone,
- au moins un capteur (3, 2) pour mesurer un signal électrique, et
- un moyen de commande (4) qui détecte l'apparition d'une caractéristique pathologique du signal électrique mesuré par le capteur (3, 2) et qui transmet une séquence de stimuli à l'électrode (2) lorsque la caractéristique pathologique se produit,
**caractérisé en ce que**
la séquence de stimuli comprend une séquence à basse fréquence de trains d'impulsions courts haute fréquence de remise à zéro suivis d'un train d'impulsions court haute fréquence à action désynchronisante, sachant que des trains d'impulsions haute fréquence de remise à zéro successifs ne se chevauchent jamais et que les trains d'impulsions haute fréquence de remise à zéro présentent respectivement une amplitude plus grande que le train d'impulsions haute fréquence à action désynchronisante.

2. Dispositif d'après la revendication 1, sachant que les trains d'impulsions à haute fréquence de remise à zéro présentent respectivement une amplitude comprise entre 3 et 7 V et que le train d'impulsions à haute fréquence à action désynchronisante présente une amplitude comprise entre 2 et 6 V.

3. Dispositif d'après la revendication 1, sachant que les trains d'impulsions à haute fréquence de remise à zéro présentent respectivement un nombre d'impulsions individuelles plus élevé que le train d'impulsions à haute fréquence à action désynchronisante.

4. Dispositif d'après la revendication 3, sachant que les trains d'impulsions à haute fréquence de remise à zéro sont composés respectivement de 4 à 20 impulsions individuelles et que le train d'impulsions à haute fréquence à action désynchronisante est composé de 3 à 15 impulsions individuelles.
